# EUROPEAN PATENT SPECIFICATION

(11) **EP 1 122 307 B1**
(45) Date of publication and mention of the grant of the patent: **22.04.2009**
(21) Application number: 00400313.3
(22) Date of filing: 04.02.2000
(51) Int. Cl.: C12N 9/48, C12N 15/63, C12N 5/10, C07K 16/40, G01N 33/50, A61K 48/00, A61K 38/48, A61P 1/00, A61P 9/00, A61P 19/00, A61P 25/00, A61P 29/00

(54) **Human carnosinase 2**
Menschliche Carnosinase 2
Carnosinase 2 humaine

(43) Date of publication of application: 08.08.2001
(73) Proprietor: Sanofi-Aventis, 75013 Paris (FR)
(72) Inventor: Ledig, Jean-Pierre, 67100 Strasbourg (FR); Saudek, Vladimir, 67300 Schiltigheim (FR)
(74) Representative: Thouret-Lemaitre, Elisabeth

(56) References cited:
- LENNEY J.F. ET AL.: "Characterization of human tissue carnosinase" BIOCHEMICAL JOURNAL, vol. 228, 1985, pages 653-660, XP000864719
- BLUM H. ET AL.: "Homo sapiens mRNA; EST DKFZp564G2472_r1 (from clone DKFZp564G2472)" EMBL DATABASE ENTRY HSM001879, ACCESSION NO. AL037548, 12 March 1999 (1999-03-12), XP002142579
- NCI-CGAP: "National Cancer Institute, Cancer Genome Anatomy Project (CGAP), Tumor Gene Index http://www.ncbi.nlm.nih.gov/ncicgap; qt63h04.x1 NCI_CGAP_Eso2 Homo sapiens cDNA clone IMAGE:1959991 3' similar to TR:P78801 P78801 FISSION YEAST;mRNA sequence" EMBL DATABASE ENTRY AI270136, ACCESSION NO. AI270136, 19 November 1998 (1998-11-19), XP002142580
- ADAMS M. D. ET AL.: "Initial Assessment of Human Gene Diversity and Expression Patterns Based Upon 83 Million Basepairs of cDNA Sequence; EST56447 Infant brain Homo sapiens cDNA 5' end similar to S. cerevisiae hypothetical protein YBR2018" EMBL DATABASE ENTRY HSZZ54755, ACCESSION NO. AA349630, 18 April 1997 (1997-04-18), XP002142581
- HILLIER L. ET AL.: "The WashU-Merck EST Project; yg92b05.r1 Soares infant brain 1NIB Homo sapiens cDNA clone IMAGE:40803 5', mRNA sequence" EMBL DATABASE ENTRY HS887124, ACCESSION NO. R55887, 28 May 1995 (1995-05-28), XP002142582
- JACKSON M. C. ET AL.: "Purification and properties of human serum carnosinase" CLINICA CHIMICA ACTA, vol. 196, no. 2-3, 1991, pages 193-205, XP000874246

### SEQUENCE LISTING

<110> SANOFI-SYNTHELABO
<120> Human carnosinase 2, its isolation and uses
<130> Human plasmid Sgc-551
<140>
   <141>
<160> 2
<170> PatentIn Ver. 2.1
<210> 1
   <211> 1428
   <212> DNA
   <213> Homo sapiens
<220>
   <221> CDS
   <222> (1)..(1428)
<400> 1
   atg gcg gcc ctc act acc ctg ttt aag tac ata gat gaa aat cag gat     48
   Met Ala Ala Leu Thr Thr Leu Phe Lys Tyr Ile Asp Glu Asn Gln Asp
    1               5                  10                  15
   cgc tac att aag aaa ctc gca aaa tgg gtg gct atc cag agt gtg tct     96
   Arg Tyr Ile Lys Lys Leu Ala Lys Trp Val Ala Ile Gln Ser Val Ser
               20                  25                  30
   gcg tgg ccg gag aag aga ggc gaa atc agg agg atg atg gaa gtt gct    144
   Ala Trp Pro Glu Lys Arg Gly Glu Ile Arg Arg Met Met Glu Val Ala
           35                  40                  45
   gct gca gat gtt aag cag ttg ggg ggc tct gtg gaa ctg gtg gat atc    192
   Ala Ala Asp Val Lys Gln Leu Gly Gly Ser Val Glu Leu Val Asp Ile
       50                  55                  60
   gga aaa caa aag ctc cct gat ggc tcg gag atc ccg ctc cct cct att    240
   Gly Lys Gln Lys Leu Pro Asp Gly Ser Glu Ile Pro Leu Pro Pro Ile
   65                  70                  75                  80
   ctg ctc ggc agg ctg ggc tcc gac cca cag aag aag acc gtg tgc att    288
   Leu Leu Gly Arg Leu Gly Ser Asp Pro Gln Lys Lys Thr Val Cys Ile
                   85                  90                  95
   tac ggg cac ctg gat gtg cag cct gca gcc ctg gag gac ggc tgg gac    336
   Tyr Gly His Leu Asp Val Gln Pro Ala Ala Leu Glu Asp Gly Trp Asp
               100                 105                 110
   agc gag ccc ttc acc ctg gtg gag cga gac ggc aag ctg tat ggg aga    384
   Ser Glu Pro Phe Thr Leu Val Glu Arg Asp Gly Lys Leu Tyr Gly Arg
           115                 120                 125
   ggt tcg act gat gat aag ggc ccg gtg gcc ggc tgg ata aac gcc ctg    432
   Gly Ser Thr Asp Asp Lys Gly Pro Val Ala Gly Trp Ile Asn Ala Leu
       130                 135                 140
   gaa gcg tat cag aaa aca ggc cag gag att cct gtc aac gtc cga ttc    480
   Glu Ala Tyr Gln Lys Thr Gly Gln Glu Ile Pro Val Asn Val Arg Phe
   145                 150                 155                 160
   tgc ctc gaa ggc atg gag gag tca ggc tct gag ggc cta gac gag ctg    528
   Cys Leu Glu Gly Met Glu Glu Ser Gly Ser Glu Gly Leu Asp Glu Leu
                   165                 170                 175
   att ttt gcc cgg aaa gac aca ttc ttt aag gat gtg gac tat gtc tgc    576
   Ile Phe Ala Arg Lys Asp Thr Phe Phe Lys Asp Val Asp Tyr Val Cys
               180                 185                 190
   att tct gac aat tac tgg ctg gga aag aag aag ccc tgc atc acc tac    624
   Ile Ser Asp Asn Tyr Trp Leu Gly Lys Lys Lys Pro Cys Ile Thr Tyr
           195                 200                 205
   ggc ctc agg ggc att tgc tac ttt ttc atc gag gtg gag tgc agc aac    672
   Gly Leu Arg Gly Ile Cys Tyr Phe Phe Ile Glu Val Glu Cys Ser Asn
       210                 215                 220
   aaa gac ctc cat tct ggg gtg tac ggg ggc tcg gtg cat gag gcc atg    720
   Lys Asp Leu His Ser Gly Val Tyr Gly Gly Ser Val His Glu Ala Met
   225                 230                 235                 240
   act gat ctc att ttg ctg atg ggc tct ttg gtg gac aag agg ggg aac    768
   Thr Asp Leu Ile Leu Leu Met Gly Ser Leu Val Asp Lys Arg Gly Asn
                   245                 250                 255
   atc ctg atc ccc ggc att aac gag gcc gtg gcc gcc gtc acg gaa gag    816
   Ile Leu Ile Pro Gly Ile Asn Glu Ala Val Ala Ala Val Thr Glu Glu
               260                 265                 270
   gag cac aag ctg tac gac gac atc gac ttt gac ata gag gag ttt gcc    864
   Glu His Lys Leu Tyr Asp Asp Ile Asp Phe Asp Ile Glu Glu Phe Ala
           275                 280                 285
   aag gat gtg ggg gcg cag atc ctc ctg cac agc cac aag aaa gac atc    912
   Lys Asp Val Gly Ala Gln Ile Leu Leu His Ser His Lys Lys Asp Ile
       290                 295                 300
   ctc atg cac cga tgg cgg tac ccg tct ctg tcc ctc cat ggc atc gaa    960
   Leu Met His Arg Trp Arg Tyr Pro Ser Leu Ser Leu His Gly Ile Glu
   305                 310                 315                 320
   ggc gcc ttc tct ggg tct ggg gcc aag acc gtg att ccc agg aag gtg    1008
   Gly Ala Phe Ser Gly Ser Gly Ala Lys Thr Val Ile Pro Arg Lys Val
                   325                 330                 335
   gtt ggc aag ttc tcc atc agg ctc gtg ccg aac atg act cct gaa gtc    1056
   Val Gly Lys Phe Ser Ile Arg Leu Val Pro Asn Met Thr Pro Glu Val
               340                 345                 350
   gtc ggc gag cag gtc aca agc tac cta act aag aag ttt gct gaa cta    1104
   Val Gly Glu Gln Val Thr Ser Tyr Leu Thr Lys Lys Phe Ala Glu Leu
           355                 360                 365
   cgc agc ccc aat gag ttc aag gtg tac atg ggc cac ggt ggg aag ccc    1152
   Arg Ser Pro Asn Glu Phe Lys Val Tyr Met Gly His Gly Gly Lys Pro
       370                 375                 380
   tgg gtc tcc gac ttc agt cac cct cat tac ctg gct ggg aga aga gcc    1200
   Trp Val Ser Asp Phe Ser His Pro His Tyr Leu Ala Gly Arg Arg Ala
   385                 390                 395                 400
   atg aag aca gtt ttt ggt gtt gag cca gac ttg acc agg gaa ggc ggc    1248
   Met Lys Thr Val Phe Gly Val Glu Pro Asp Leu Thr Arg Glu Gly Gly
                   405                 410                 415
   agt att ccc gtg acc ttg acc ttt cag gag gcc acg ggc aag aac gtc    1296
   Ser Ile Pro Val Thr Leu Thr Phe Gln Glu Ala Thr Gly Lys Asn Val
           420                 425                 430
   atg ctg ctg cct gtg ggg tca gcg gat gac gga gcc cac tcc cag aat    1344
   Met Leu Leu Pro Val Gly Ser Ala Asp Asp Gly Ala His Ser Gln Asn
       435                 440                 445
   gaa aag ctc aac agg tat aac tac ata gag gga acc aag atg ctg gcc    1392
   Glu Lys Leu Asn Arg Tyr Asn Tyr Ile Glu Gly Thr Lys Met Leu Ala
   450                 455                 460
   gcg tac ctg tat gag gtc tcc cag ctg aag gac tag                    1428
   Ala Tyr Leu Tyr Glu Val Ser Gln Leu Lys Asp
   465                 470                 475
<210> 2
   <211> 475
   <212> PRT
   <213> Homo sapiens
<400> 2
   Met Ala Ala Leu Thr Thr Leu Phe Lys Tyr Ile Asp Glu Asn Gln Asp
   1               5                   10                  15
   Arg Tyr Ile Lys Lys Leu Ala Lys Trp Val Ala Ile Gln Ser Val Ser
               20                  25                  30
   Ala Trp Pro Glu Lys Arg Gly Glu Ile Arg Arg Met Met Glu Val Ala
           35                  40                  45
   Ala Ala Asp Val Lys Gln Leu Gly Gly Ser Val Glu Leu Val Asp Ile
       50                  55                  60
   Gly Lys Gln Lys Leu Pro Asp Gly Ser Glu Ile Pro Leu Pro Pro Ile
   65                  70                  75                  80
   Leu Leu Gly Arg Leu Gly Ser Asp Pro Gln Lys Lys Thr Val Cys Ile
                   85                  90                  95
   Tyr Gly His Leu Asp Val Gln Pro Ala Ala Leu Glu Asp Gly Trp Asp
               100                 105                 110
   Ser Glu Pro Phe Thr Leu Val Glu Arg Asp Gly Lys Leu Tyr Gly Arg
   115 120 125
   Gly Ser Thr Asp Asp Lys Gly Pro Val Ala Gly Trp Ile Asn Ala Leu
   130 135 140
   Glu Ala Tyr Gln Lys Thr Gly Gln Glu Ile Pro Val Asn Val Arg Phe
   145 150 155 160
   Cys Leu Glu Gly Met Glu Glu Ser Gly Ser Glu Gly Leu Asp Glu Leu
   165 170 175
   Ile Phe Ala Arg Lys Asp Thr Phe Phe Lys Asp Val Asp Tyr Val Cys
   180 185 190
   Ile Ser Asp Asn Tyr Trp Leu Gly Lys Lys Lys Pro Cys Ile Thr Tyr
   195 200 205
   Gly Leu Arg Gly Ile Cys Tyr Phe Phe Ile Glu Val Glu Cys Ser Asn
   210 215 220
   Lys Asp Leu His Ser Gly Val Tyr Gly Gly Ser Val His Glu Ala Met
   225 230 235 240
   Thr Asp Leu Ile Leu Leu Met Gly Ser Leu Val Asp Lys Arg Gly Asn
   245 250 255
   Ile Leu Ile Pro Gly Ile Asn Glu Ala Val Ala Ala Val Thr Glu Glu
   260 265 270
   Glu His Lys Leu Tyr Asp Asp Ile Asp Phe Asp Ile Glu Glu Phe Ala
   275 280 285
   Lys Asp Val Gly Ala Gln Ile Leu Leu His Ser His Lys Lys Asp Ile
   290 295 300
   Leu Met His Arg Trp Arg Tyr Pro Ser Leu Ser Leu His Gly Ile Glu
   305 310 315 320
   Gly Ala Phe Ser Gly Ser Gly Ala Lys Thr Val Ile Pro Arg Lys Val
   325 330 335
   Val Gly Lys Phe Ser Ile Arg Leu Val Pro Asn Met Thr Pro Glu Val
                   340                 345                 350
   Val Gly Glu Gln Val Thr Ser Tyr Leu Thr Lys Lys Phe Ala Glu Leu
           355                 360                 365
   Arg Ser Pro Asn Glu Phe Lys Val Tyr Met Gly His Gly Gly Lys Pro
       370                 375                 380
   Trp Val Ser Asp Phe Ser His Pro His Tyr Leu Ala Gly Arg Arg Ala
   385                 390                 395                 400
   Met Lys Thr Val Phe Gly Val Glu Pro Asp Leu Thr Arg Glu Gly Gly
                   405                 410                 415
   Ser Ile Pro Val Thr Leu Thr Phe Gln Glu Ala Thr Gly Lys Asn Val
               420                 425                 430
   Met Leu Leu Pro Val Gly Ser Ala Asp Asp Gly Ala His Ser Gln Asn
           435                 440                 445
   Glu Lys Leu Asn Arg Tyr Asn Tyr Ile Glu Gly Thr Lys Met Leu Ala
       450                 455                 460
   Ala Tyr Leu Tyr Glu Val Ser Gln Leu Lys Asp
   465                 470                 475

## Description

The invention relates to newly identified polynucleotides, to polypeptides encoded by them and to the use of such polynucleotides and polypeptides. The invention also relates to their production. More particularly, the polynucleotides and polypeptides of the invention relate to human carnosinase 2, hereinafter referred to as HC2. The invention also relates to inhibiting or activating the action of such polynucleotides and polypeptides.

Carnosine (b-alanyl-L-histidine) and structurally related dipeptides such as homocarnosine (g-aminobutyryl-L-histidine) and anserine (b-alanyl-L-1-methylhistidine) represent a group of histidine-containing molecules widely distributed in vertebrate organisms, and are particularly abundant in muscles and nervous tissues. Carnosine has been found also in some molluscs and arthropods (Comp. Biochem. Physiol. 1970 34: 3-30). The concentration of carnosine in the human skeletal muscle has been shown to be very high - up to 20 mM - in comparison with other mammals.

The exact biological functions of the aminoacyl-histidine dipeptides are still unknown but they are believed to act as cytosolic buffering agents. Numerous studies have demonstrated, both at the tissue and organelle levels, that they possess strong and specific antioxidant properties, by preventing the accumulation of oxidised products derived from the lipid peroxidation of biological membranes (Int. J. Biochem. 1993 25 : 1101-1107). Carnosine has been shown to act as a competitive inhibitor of the non-enzymatic glycosylation of proteins (FEBS Lett. 1995 371 : 81-85). Thus, carnosine may prevent the formation of the advanced glycation end-products (AGEs), whose accumulation in the brain has been proposed to play a direct role in the etiology and pathogenesis of Alzheimer's disease. Other roles ascribed to these dipeptides include actions as neurotransmitters and chelation of heavy metals. Accordingly, many therapeutic actions of carnosine and histidine-containing dipeptides have been suggested, including antihypertensive effects, actions as immunomodulating agents, wound healing and antineoplastic effects (Mol Aspects Med 1992;13(5):379-444).

A carnosinase and an homocarnosinase responsible for the degradation of carnosine have been identified in different tissues (Clin Chim Acta 1991 196 (2-3):193-205; Archs. Biochem. Biophys. 1960 88: 83-93; Biochim. Blophys. Acta. 1976 429: 214-219 Biochemical Journal, 1985, 228, 653-660). Two types of carnosinases were reported, i.e. tissue and plasma carnosinases. In contrast to human plasma carnosinase, human tissue carnosinase does not hydrolyse anserine or homocarnosine and probably has little or no activity against carnosine *in vivo,* because of its high pH optimum (9.5) and its high Km value. Human serum carnosinase is found to be a glycoprotein with an isoelectric point of 4.4 and a subunit molecular weight of 75.000; the active enzyme being a dimer and the two subunits being connected by one or more disulphide bonds. Human serum carnosinase was suggested to be synthesized in the brain and secreted into cerebrospinal fluid (CSF), which carries it into the blood stream. The physiological function of this enzyme seems to be the hydrolysis of homocarnosine in the brain and the splitting of carnosine and anserine in the blood stream. Several reports have shown that the level of carnosinase in the blood increases with age, reaching an adult level at ages varying from 12 to 15 years.

Patients affected with homocarnosinosis suffer from progressive dementia, spastic paraplegia and abnormal retinal pigmentation and present greatly elevated concentrations of homocarnosine in the brain and CSF. Several reports describe variable phenotypes for a serum carnosinase deficiency, which range from normal to severe psychomotor retardation, hypotonia, and myoclonic seizures in the first year of life.

Two unrelated children with a progressive neurologic disorder characterized by severe mental defect and myoclonic seizures have been described (Lancet I: 1229-1230, 1968). Both excreted carnosine in the urine, even when any source of the dipeptide is excluded from the diet. Both had unusually high concentrations of homocarnosine in the cerebrospinal fluid. It was suggested that one of the children, and perhaps both, presented a defect in carnosinase activity, this enzyme being almost absent in the two patients. Autopsy on one of the boy (7-year old), whose parents had a low carnosinase activity, showed severe axonal degeneration, numerous 'spheroids' in the grey matter, demyelinization, fibrosis, and loss of Purkinje fibers (Neurology 22: 644-654, 1972).

Serum carnosinase activity is shown to be altered in patients with neurological disorders. Reduced serum carnosinase activity has been observed in patients with Parkinson's disease and multiple sclerosis compared with a control group. Carnosinase activity, 5-10% of that found in serum, was detected in CSF samples (Clin. Chim. Acta 1994 Feb;225(1):57-64). The cause of reduced serum carnosinase activities in central nervous system disorders remains unclear.

This indicates that human carnosinase has an established, proven history as therapeutic target and that it can play a role in preventing, ameliorating or correcting dysfunctions or diseases, including, but not limited to, cognitive disorders, developmental abnormalities and fetal deficiencies, neurodegenerative disorders such as amyotrophic lateral sclerosis, Parkinson's disease, schizophrenia, abnormal mental states, ischaemic shock, epilepsia, polyarthritis, hypertension, convulsions, ischaemic heart damage, ulcers, adrenal cortical function, wound healing, trauma, inflammatory diseases.

In one aspect, the invention relates to HC2 polypeptides and polynucleotides, and to recombinant materials and methods for their production. Another aspect of the invention relates to methods for using such HC2 polypeptides and polynucleotides, for example in the treatment of cognitive disorders, developmental abnormalities and fetal deficiencies, neurodegenerative disorders such as amyotrophic lateral sclerosis, Parkinson's disease, schizophrenia, abnormal mental states, ischaemic shock, epilepsia, polyarthritis, hypertension, convulsions, ischaemic heart damage, ulcers, adrenal cortical function, wound healing, trauma, inflammatory diseases, among others. In still another aspect, the invention relates to methods to identify HC2 activators and inhibitors using the materials provided by the invention, and treating conditions associated with the use of such compounds. Yet another aspect of the invention relates to diagnostic assays for detecting diseases associated with inappropriate HC2 activity or levels.
Figure 1 (A and B) is a histogram showing the relative abundance of the messenger RNA of HC2 in different human tissues.
Figure 2 is a histogram showing the enzymatic activity of carnosinase measured by micro-fluorescence in transfected CHO cells.

The following definitions are provided to facilitate understanding of certain terms used frequently herein.

"HC2" refers to a polypeptide comprising the amino acid sequence set forth in SEQ ID NO:2, or an allelic variant thereof, as well as other variants as described further.

"HC2 activity" or "biological activity of HC2" refers to the metabolic or physiologic function of said HC2, including similar activities or improved activities or these activities with decreased undesirable side-effects. Also included are antigenic and immunogenic activities of HC2.

"HC2 gene" or "HC2 polynucleotide" refers to a polynucleotide comprising the nucleotide sequence set forth in SEQ ID NO:1 or allelic variants thereof and/or their complements, as described in the following specification.

"Antibodies" as used herein include polyclonal and monoclonal antibodies, chimeric, single chain, and humanized antibodies (an antibody - mouse or any other - with some specific parts changed to human homologous regions), as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein. For example, recombinant DNA molecules contained in a vector are considered isolated for the purposes of the invention. Further examples of isolated nucleic acid molecules include recombinant DNA molecules maintained in heterologous host cells or purified DNA molecules in solution. Isolated RNA molecules include *in vivo* or *in vitro* RNA transcripts of the DNA molecules of the invention. Isolated nucleic acid molecules according to the invention further include such molecules produced synthetically.

"Polynucleotide" generally refers to any polyribonucleotide or polydeoxribonucleotide, which may be unmodified RNA or DNA or modified RNA or DNA. "Polynucleotides" include, without limitation, single- and double-stranded DNA, DNA that is a mixture of single- and double-stranded regions, single- and double-stranded RNA, and RNA that is a mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNA or RNA containing one or more modified bases and DNA or RNA with backbones modified for stability or for other reasons as explained in the present application. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. Thus, "polynucleotide" also encompasses variants of the nucleic acid molecules of the invention which encode portions, analogs or derivatives of HC2. Variants may occur naturally, such as natural allelic variants. By an "allelic variant" is intended one of several alternate forms of a gene occupying a given locus on a chromosome of an organism. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, Proteins - Structure and molecular properties, 2nd Ed., T. E. Creighton, W. H. Freeman and Company, New York, 1993 and F. Wold, Post-translational Protein Modifications: Perspectives and Prospects, p. 1-12 in Post-translational covalent modification of proteins, B. C. Johnson, Ed., Academic Press, New York, 1983; Seifter et al., "Analysis for protein modifications and non-protein cofactors", Meth. Enzymol. (1990) 182:626-646 and Rattan et al., "Protein Synthesis: Post-translational Modifications and Aging", Ann. NY Acad. Sci. (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be naturally occurring, such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g. : Computational Molecular Biology, Lesk, A.M., ed., Oxford University Press, New York, 1988; Biocomputing Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991. While there exists a number of methods to measure identity between two polynucleotide or polypeptide sequences, the term "identity" is well known to skilled artisans (Carillo, H., and Lipton, D., SIAM J. Applied Math. (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., SIAM J. Applied Math. (1988) 48:1073. Methods to determine identity and similarity are codified in computer programs. Preferred computer program methods to determine identity and similarity between two sequences include, but are not limited to, GCS program package (Devereux, J., et al., Nucleic Acids Research (1984) 12(1):387), BLASTP, BLASTN, FASTA (Atschul, S.F. et al., J. Molec. Biol. (1990) 215:403).

As an illustration, by a polynucleotide having a nucleotide sequence having at least, for example, 95% "identity" to a reference nucleotide sequence is intended a polynucleotide in which the nucleotide sequence is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence is intended a polypeptide having an amino acid sequence identical to the reference sequence except that the polypeptide sequence may include up to five amino acid alterations per each 100 amino acids of the reference amino acid. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

In one aspect, the invention relates to isolated HC2 polypeptides. The HC2 polypeptides include the polypeptide of SEQ ID NO:2; as well as polypeptides comprising the amino acid sequence of SEQ ID NO:2; and polypeptides comprising an amino acid sequence which has at least 80% identity to that of SEQ ID NO:2 over its entire length, and still more preferably at least 90% identity, and even still more preferably at least 95% identity to SEQ ID NO: 2. Furthermore, those with at least 97-99% identity are highly preferred. Also included within HC2 polypeptides are polypeptides having the amino acid sequence which have at least 80% identity to the polypeptide having the amino acid sequence of SEQ ID NO: 2 over its entire length, and still more preferably at least 90% identity, and even still more preferably at least 95% identity to SEQ ID NO: 2. Furthermore, those with at least 97-99% identity are highly preferred.

It will be recognized that some amino acids of the HC2 polypeptides can be varied without significant effect on the structure or function of the protein. Guidance concerning with amino acid changes which are likely to be phenotypically silent can be found in Bowie, J. U. et al, "Deciphering the Message in Protein Sequences : Tolerance to Amino Acid Substitutions", Science 247 : 1306-1310 (1990).

Thus, the invention further provides variants of the HC2 polypeptides which show substantial HC2 polypeptide activity or which include regions of HC2 protein such as the protein portions discussed below. Such mutants include HC2 polypeptides in which deletions, insertions, type substitutions, inversions and/or repeats have occurred.

Thus, the fragment, derivative or analog of the polypeptide of SEQ ID NO:2, or that encoded by the deposited cDNA, may be :
- one in which one or more of the amino acid residues are substituted with a conserved or non-conserved amino acid residue, preferably a conserved amino acid residue, and such substituted amino acid residue may or may not be one encoded by the genetic code, or
- one in which one or more of the amino acid residues includes a substituent group, or
- one in which the mature polypeptide is fused with another compound, such as a compound for increasing the half-life of the polypeptide (for example, polyethylene glycol), or
- one in which additional amino acids are fused to the mature polypeptide, such as the IgG Fc fusion region peptide or leader or secretory sequence which is employed for purification of the mature polypeptide or a pro-protein sequence. Such fragments, derivatives and analogs are deemed to be within the scope of the invention.

Of particular interest are substitutions of charged amino acids with another charged amino acid and with neutral or negatively charged amino acids. The latter results in proteins with reduced positive charge to improve the characteristics of the HC2 protein, for example for prevention of aggregation.

As indicated, changes are preferably of a minor nature, such as conservative amino acid substitutions that do not significantly affect the activity of the protein, such as those indicated hereafter.

| | |
|---|---|
| Aromatic | Phenylalanine |
| | Tryptophan |
| | Tyrosine |
| Hydrophobic | Leucine |
| | Isoleucine |
| | Valine |
| Polar | Glutamine |
| | Asparagine |
| Basic | Arginine |
| | Lysine |
| | Histidine |
| Acidic | Aspartic acid |
| | Glutamic acid |
| Small | Alanine |
| | Serine |
| | Threonine |
| | Methionine |
| | Glysine |

Generally, the number of amino acid substitutions will not be more than 50.

Amino acids in the HC2 polypeptide of the invention that are essential for function can be identified by methods known in the art, such as site-directed mutagenesis or alanine-scanning mutagenesis (Cunnigham and Wells, Science 244 : 1081-1085 (1989)). The latter procedure introduces single alanine mutations at every residue in the molecule. The resulting mutant molecules are then tested for biological activity such as receptor binding or *in vitro* proliferative activity. Sites that are critical for ligand-receptor binding can also be determined by structural analysis, e.g. crystallization, nuclear magnetic resonance or photoaffinity labeling (Smith et al., J. Mol. Biol. 224 : 899-904 (1992); de Vos et al., Science 255 : 306-312 (1992)).

As to the selection of peptides or polypeptides bearing an antigenic epitope, it is well known that relatively short synthetic sequences that mimic part of a protein sequence are routinely capable of eliciting an antiserum that reacts with the partially mimicked protein. See for instance Sutcliffe, J. G., Shinnick, T. M. , Green, N. and Learner, R. A., Antibodies that React with Predetermined Sites on Proteins, Science 219 : 660-666 (1983). Peptides capable of eliciting protein-reactive sera are frequently represented in the primary sequence of a protein, can be characterized by a set of simple chemical rules, and are confined neither to immunodominant regions of intact proteins (i.e. immunogenic epitopes) nor to the amino or carboxyl terminals.

Antigenic epitope-bearing peptides and polypeptides of the invention are therefore useful to raise antibodies, including monoclonal antibodies, that bind specifically to a polypeptide of the invention. Antigenic epitope-bearing peptides and polypeptides of the invention preferably contain a sequence of at least seven, more preferably at least nine and most preferably between about 15 to about 30 amino acids contained within the amino acid sequence of a polypeptide according to the invention.

For instance, antigenic polypeptides or peptides that can be used to generate HC2 specific antibodies comprise : a polypeptide comprising amino acid residues from about 445 to about 460 of the amino acid sequence set forth in SEQ ID NO:2, a polypeptide comprising amino acid residues from about 10 to about 25 of the amino acid sequence set forth in SEQ ID NO:2.

The epitope-bearing peptides and polypeptides of the invention can be prepared by any conventional means, for instance by the method described in document US Patent n° 4,631,211.

HC2 polypeptides of the invention and the epitope-bearing fragments thereof can be combined with parts of the constant domain of immunoglobulins (IgG), resulting in chimeric polypeptides. These fusion proteins facilitate the purification and generally show an increased half life *in vivo.* See for instance Traunecker et al., Nature 331 : 84-86 (1988). Fusion proteins that have a disulfide-linked dimeric structure due to the IgG part can also be more efficient in binding and neutralizing other molecules than the monomeric HC2 protein or protein fragment thereof (Fountoulakis et al., J. Biochem. 270 : 3958-3964 (1995)).

The HC2 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the HC2 polypeptides or variants, mutated forms, derivatives thereof as hereabove mentioned are also included in the invention. A fragment is a polypeptide having an amino acid sequence that entirely is the same as part, but not all, of the amino acid sequence of the aforementioned HC2 polypeptides. As with HC2 polypeptides, fragments may be "free-standing," or comprised within a larger polypeptide of which they form a part or region, most preferably as a single continuous region. Representative examples of polypeptide fragments of the invention, include, for example, fragments from about amino acid numbers 81-100, and 101 to the end of the HC2 polypeptide set forth in SEQ ID NO:2. In this context "about" includes the particularly recited ranges larger or smaller by several, for example 5, 4, 3, 2 or 1 amino acid at either extreme or at both extremes.

Preferred fragments include, for example, truncation polypeptides having the amino acid sequence of HC2 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, tum and tum-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate HC2 activity, including those with a similar activity or an improved activity, or with a decreased undesirable activity. Also included are those that are antigenic or immunogenic in an animal, especially in a human.

The HC2 polypeptides of the invention can be prepared in any suitable manner. Such polypeptides include isolated naturally occurring polypeptides, recombinantly produced polypeptides, or polypeptides produced by a combination of these methods.

In one embodiment, the HC2 polypeptides of the invention can be prepared by chemical synthesis, for example by using non natural and/or modified amino acids. Thus, it can be advantageous to use non natural amino acids, for example D-amino acids, to enhance life time of the polypeptides, or analogs of amino acids, for example sulfurized forms of amino acids.

Methods for preparing such polypeptides are known in the art.

For example, chemical synthesis of the polypeptides of the invention can be carried out by using a solid phase process. In such method, the C-terminal amino acid is fixed onto an inert solid support et comprises protective groups (e.g. BOC or FMOC) of the alpha-amino moiety. At the end of this step, the protective group is removed and a second amino acid, optionally protected in a similar manner, is fixed. The N-terminal protective groups are removed after each amino acid is fixed, protective groups possibly present on the lateral chains being conserved. When the peptide chain is synthesized, the peptide is cleaved from the support and the remaining protective groups are removed. Coupling can be carried out by using N,N'-diisopropyl-carbodiimine (DIC), among others, in a solvent of DMF or DCM. This solid phase synthesis is described in for example Stewart J. M. et al., Solid Phase Peptides Synthesis. Pierce Chem. Company, Rockford, 111, 2nd Ed., (1984).

Other polypeptides according to the invention comprise the amino acid sequence of the HC2 polypeptide which possesses the complete amino acid sequence encoded by the cDNA clone contained in ATCC deposit number PTA-1045 as discussed below, as well as polypeptides which comprise the amino acid sequence of the mature HC2 polypeptide which possesses the complete amino acid sequence encoded by the cDNA clone contained in ATCC deposit number PTA-1045.

In another aspect, the invention provides a peptide or polypeptide comprising an epitope-bearing portion of a polypeptide of the invention. The epitope of this polypeptide portion is an immunogenic or antigenic epitope of a polypeptide described in the invention. An immunogenic epitope is defined as a part of a protein that elicits an antibody response when the whole protein is the immunogen. On the other hand, a region of a protein molecule to which an antibody can bind is defined as an antigenic epitope.

Another aspect of the invention relates to HC2 polynucleotides
with the proviso that the polynucleotide has not one of the following sequences: and

Unless otherwise indicated, all nucleotide sequences determined by sequencing a DNA molecule herein have been determined by using an automated DNA sequencer, and all amino acid sequences of polypeptides encoded by DNA sequences determined herein have been predicted by translation of a DNA sequence determined as above. Therefore, as is known in the art for any DNA sequence determined by this approach, any nucleotide sequence may contain some errors. Nucleotide sequences determined by automation are typically at least about 90 % identical, more typically at least about 95 % to about 99.9 % identical to the actual nucleotide sequence of the sequenced DNA molecule.

HC2 polynucleotides include Isolated polynucleotides which encode the HC2 polypeptides and fragments, and polynucleotides closely related thereto, e.g. variants, derivatives, mutated forms. More specifically, HC2 polynucleotides of the invention include a polynucleotide comprising the nucleotide sequence set forth in SEQ ID NO:1 encoding a HC2 polypeptide of SEQ ID NO: 2, and a polynucleotide having the particular sequence of SEQ ID NO:1. HC2 polynucleotides further include a polynucleotide comprising a nucleotide sequence that has at least 80% identity to a nucleotide sequence encoding the HC2 polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95% identity are especially preferred. Furthermore, those with at least 97% identity are highly preferred and those with at least 98-99% identity are most highly preferred, those with at least 99% identity being the most preferred. Also included under HC2 polynucleotides are nucleotide sequences which have sufficient identity to the nucleotide sequence contained in SEQ ID NO:1 or contained in the cDNA insert of the plasmid deposited with the ATCC Deposit number PTA-1045 discussed below to hybridize under conditions useable for amplification or for use as a probe or marker. Moreover, HC2 polynucleotides include a nucleotide sequence having at least 80% identity to a nucleotide sequence encoding the HC2 polypeptide, either in a mature form or not, expressed by the cDNA insert deposited at the ATCC with Deposit Number PTA-1045, and a nucleotide sequence comprising at least 15 contiguous nucleotides of such cDNA insert. In this regard, polynucleotides at least 90% identical are particularly preferred, and those with at least 95% identity are especially preferred. Furthermore, those with at least 97% identity are highly preferred and those with at least 98-99% identity are most highly preferred, those with at least 99% identity being the most preferred. The invention also provides polynucleotides which are complementary to all the HC2 polynucleotides according to the invention.

A deposit containing a human HC2 cDNA has been deposited with the American Type Culture Collection (ATCC), 10801 University Bld, Manassas, VA 20110-2209, USA, on December 10, 1999, and assigned ATCC Deposit Number PTA-1045. The deposited material (clone) is plasmid pCMVSport3.0 that further contains the full length cDNA coding for HC2, referred to as "human plasmid Sgc-551" upon deposit. The cDNA insert is within Sal1 and Not1 sites in the vector. The nucleotide sequence of the polynucleotide contained in the deposited material, as well as the amino acid sequence of the polypeptide encoded thereby, are controlling in the event of any conflict with any description of sequences herein.

The deposit has been made under the terms of the Budapest Treaty on the international recognition of the deposit of micro-organisms for purposes of patent procedure.

HC2 of the invention is structurally related to other proteins of the human carnosinase family, as shown by the results of sequencing the cDNA of SEQ ID NO:1 encoding human HC2. The cDNA sequence of SEQ ID NO:1 contains an open reading frame (nucleotide numbers 1 to 1428) encoding a polypeptide of 475 amino acid residues of SEQ ID NO:2, with a deduced molecular weight of about 52 kDa. Amino acid sequence of SEQ ID NO:2 has about 54% identity (using Megalign with the Clustal algorithm from DNASTAR) in 466 amino acid residues with a yeast hypothetical 52.9 KD protein (CAB07646).

The polynucleotides of the invention encoding HC2 may be obtained using standard cloning and screening, from a cDNA library derived from mRNA in cells of human brain using the expressed sequence tag (EST) analysis (Adams, M.D., et al. Science (1991) 252:1651-1656; Adams, M. D. et al., Nature, (1992) 355:632-634; Adams, M. D., et al., Nature (1995) 377 Supp:3-174). Polynucleotides of the invention can also be obtained from natural sources such as genomic DNA libraries or can be synthesized using well known and commercially available techniques.

The nucleotide sequence encoding HC2 polypeptide of SEQ ID NO:2 may be identical to the polypeptide encoding sequence contained in SEQ ID NO:1 (nucleotide numbers 1 to 1428), or it may be a different sequence, which as a result of the redundancy (degeneracy) of the genetic code, also encodes the polypeptide of SEQ ID NO:2.

As indicated, the invention also provides the mature form of the HC2 polypeptide of the invention. According to the signal hypothesis, proteins secreted by mammalian cells have a signal or secretory leader sequence which is cleaved from the mature protein once export of the growing protein chain across the rough endoplasmic reticulum has been initiated. Most mammalian cells and even insect cells cleave secreted proteins with the same specificity. However, in some cases, cleavage of a secreted protein is not entirely uniform, which results in two or more mature species of the protein. Further, it has long been known that the cleavage specificity of a secreted protein is ultimately determined by the primary structure of the complete protein, i.e. its amino acid sequence. Therefore, the invention provides a nucleotide sequence encoding the mature HC2 polypeptide having the amino acid sequence encoded by the cDNA clone contained in the host identified as ATCC Deposit PTA-1045. By the mature HC2 polypeptide having the amino acid sequence encoded by the cDNA clone contained in the host identified as ATCC Deposit PTA-1045 is meant the mature form of the HC2 polypeptide produced by expression in a mammalian cell of the complete open reading frame encoded by the human DNA sequence of the clone contained in the vector in the deposited host. As indicated, the mature HC2 polypeptide having the amino acid sequence encoded by the cDNA clone contained in ATCC Deposit PTA-1045 may or may not differ from the predicted "mature" HC2 protein represented in SEQ ID NO:2, depending on the accuracy of the predicted cleavage site based on computer analysis.

Methods for predicting whether a protein has a secretory leader as well as the cleavage site of this leader sequence are available. For instance, the methods of McGeoch (Virus Res. 3:271-286 (1985)) and von Heinje (Nucleic Acids Res. 14:4683-4690 (1986)) can be used.

When the polynucleotides of the invention are used for the recombinant production of HC2 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz et al., Proc. Natl. Acad. Sci. USA (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding HC2 variants comprising the amino acid sequence of HC2 polypeptide of SEQ ID NO:2 in which several, for example 5-10, 1-5, 1-3, 1-2 or 1 amino acid residues are substituted, deleted or added, in any combination.

The invention is further directed to fragments of the isolated nucleic acid molecules described herein. By a fragment of an isolated nucleic acid molecule having the nucleotide sequence of the deposited cDNA or the nucleotide molecule having the nucleotide sequence shown in SEQ ID NO:1 is intended fragments of at least about 15 nucleotides, and preferably of at least about 20 nucleotides, more preferably of at least about 30 nucleotides, even more preferably of at least about 40 nucleotides, which are useful as diagnostic probes and primers. By a fragment of at least 20 nucleotides in length, for example, are intended fragments which include 20 or more contiguous bases from the nucleotide sequence of the deposited cDNA or from the sequence shown in SEQ ID NO:1, deleted from a continuous series of residues including the 5' end or 3' end or both.

Preferred nucleic acid fragments according to the invention comprise nucleic acid molecules which encode a polypeptide comprising amino acid residues from about 1 to about 150 of the amino acid sequence shown in SEQ ID NO:2, nucleic acid molecules which encode a polypeptide comprising amino acid residues from about 151 to about 300 of the amino acid sequence of SEQ ID NO:2, nucleic acid molecules which encode a polypeptide comprising amino acid residues from about 300 to about 475 of the amino acid sequence of SEQ ID NO:2.

The invention further relates to polynucleotides that hybridize to the herein above-described sequences. In this regard, the invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof, or to the cDNA insert in the plasmid deposited at the ATCC with Deposit Number PTA-1045 or a fragment thereof, may be used as hybridization probes for cDNA and genomic DNA, to isolate full-length cDNAs and genomic clones encoding HC2 and to isolate cDNA and genomic clones of other genes that have a high sequence similarity to the HC2 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, obtaining a polynucleotide encoding HC2 polypeptide comprises the steps of screening an appropriate library under stringent hybridization conditions with a labeled probe having the SEQ ID NO: 1 or a fragment thereof; and isolating full-length cDNA and genomic clones containing said polynucleotide sequence. Such hybridization techniques are well known to those of skill in the art. Stringent hybridization conditions are as defined above or alternatively conditions under overnight incubation at 42°C in a solution comprising: 50% formamide, 5xSSC (150 mM NaCI, 15 mM trisodium citrate), 50 mM sodium phosphate (pH 7.6), 5x Denhardt's solution, 10 % dextran sulfate, and 20 µg/ml denatured, sheared salmon sperm DNA, followed by washing the filters in 0.1x SSC at about 65°C.

The polynucleotides and polypeptides of the invention may be employed as research reagents and materials for discovery of treatments and diagnostics for animal and human diseases.

The invention also relates to recombinant vectors which comprise a polynucleotide or polynucleotides according to the invention and expression system capable of producing the HC2 polypeptide when said expression system is present in a compatible host cell, host cells which are genetically engineered with vectors of the invention. The invention also relates to the production of polypeptides of the invention by recombinant techniques. Cell-free translation systems can also be employed to produce such proteins using RNAs derived from the DNA constructs of the invention.

For recombinant production, host cells can be genetically engineered to incorporate expression systems or portions thereof for polynucleotides of the invention. Introduction of polynucleotides into host cells can be effected by methods described in many standard laboratory manuals, such as Davis et al., Basic Methods in Molecular Biology (1986) and Sambrook et al., Molecular Cloning : a Laboratory Manual, 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

The expression vectors will preferably include at least one selectable marker. Such markers include dihydrofolate reductase or neomycin resistance for eukaryotic cell culture and tetracycline or ampicillin resistance genes for culturing *E. coli* and other bacteria.

The DNA insert should preferably be linked to an appropriate promoter, such as the bacterial phage lambda PL and RP, T3 and T7, *E. coli lac*, *trp* and *tac* promoters, the eukaryotic SV40 early and late promoters, CMV immediate early promoters, eukaryotic promoters from retroviral LTRs, such as those of the Rous Sarcoma Virus (RSV) to name a few. Other suitable promoters are available for the skilled artisan.

The expression constructs will further contain sites for transcription initiation, termination and, in the transcribed region, a ribosome binding site for translation. The coding region of the mature transcripts expressed by the constructs will preferably include a translation initiating at the beginning and a termination codon (UAA, AGA or UAG) appropriately positioned at the end of the polypeptide to be translated.

Representative examples of appropriate hosts comprise bacterial cells, such as streptococci, staphylococci, *E. coli, Streptomyces, Salmonella typhimurium* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. Generally, any system or vector suitable to maintain, propagate or express polynucleotides, for producing a polypeptide in a host, may be used.

Among vectors preferred for use in bacteria are pGE70, pGE60 and pG-9, available form Qiagen; Phagescript vectors, pBS vectors, Bluescript vectors, pNH8A, pNH16a, pNH18A, pNH46A, available from Stratagene, ptrc99a, pKK223-3, pDR540, pRIT5 available from Pharmacia.

Among preferred eukaryotic vectors are pWLNEO, pSV2CAT, pOG44, pXT1 and pSG available from Stratagene; pSVK3, pBPV, pMSG and pSVL available from Pharmacia, pCDNA3, pCDNA3.1 and pTracer^{™} available from Invitrogene. Other suitable vectors will be readily apparent to the skilled in the art.

The appropriate nucleotide sequence may be inserted into an expression system by any of a variety of well-known and routine techniques, such as, for example, those set forth in Sambrook *et al*., *Molecular Cloning, a Laboratory Manual (supra).*

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the HC2 polypeptide is to be expressed for use in screening assays, generally, it is preferred that the polypeptide be produced at the surface of the cell. In this event, the cells may be harvested prior to use in the screening assay. If HC2 polypeptide is secreted into the medium, the medium can be recovered in order to recover and purify the polypeptide; if produced intracellularly, the cells must first be lysed before the polypeptide is recovered.

HC2 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

The polypeptide may be expressed in a modified form, such as a fusion protein, and may include not only secretion signals, but also additional heterologous functional regions. For instance, a region of additional amino acids, particularly charged amino acids, may be added to the N-terminus of the polypeptide to improve stability and persistence in the host cell, during purification or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to solubilize proteins. For example, EP-A-0 464 533 discloses fusion proteins comprising various portions of constant region of immunoglobulin molecules together with another human protein or part thereof. In many cases, the Fc part in a fusion protein is advantageous for use in therapy and diagnosis. On the other hand, for some uses, it would be desirable to delete the Fc part after the fusion protein has been expressed, detected and purified. For instance, it is the case when Fc portion proves to be a hindrance to use in therapy and diagnosis, for example when the fusion protein is to be used as antigen for immunizations.

It is deemed that mammals with certain diseases or susceptibility to certain diseases express significantly altered, enhanced or diminished levels of HC2 protein and mRNA encoding HC2 protein, when compared to a corresponding "standard" mammal, i.e. a mammal of the same species not having or not presenting a susceptibility to the disease. Further, it is deemed that significantly altered, enhanced or diminished levels of HC2 protein can be detected in certain body fluids (e.g. bone marrow, lymph fluid, blood, sera, plasma, saliva, urine, synovial fluid and spinal fluid) from mammals with the disease or presenting a susceptibility to the disease when compared to sera from mammals of the same species not having or not presenting a susceptibility to the disease. Thus, the invention provides a diagnostic method which involves assaying the expression level of the gene encoding HC2 of the invention in mammalian cells or body fluid and comparing the gene expression level with a standard HC2 expression level, whereby an altered, enhanced or diminished expression level of the gene with respect to the standard is indicative of the disease or of a susceptibility to the disease. Preferred mammals include monkeys, apes, cats, dogs, cows, pigs, horses, rabbits and humans.

Diseases which can be diagnosed include but are not limited to cognitive disorders, developmental abnormalities and fetal deficiencies, neurodegenerative disorders such as amyotrophic lateral sclerosis, Parkinson's disease, schizophrenia, abnormal mental states, ischaemic shock, epilepsia, polyarthritis, hypertension, convulsions, ischaemic heart damage, ulcers, adrenal cortical function, wound healing, trauma, inflammatory diseases.

By "assaying the expression level of the gene encoding HC2 " is intended qualitatively or quantitatively measuring or estimating the level of HC2 protein or the level of the mRNA encoding HC2 in a first biological sample either directly (e.g. by determining or estimating absolute protein level or mRNA level) or relatively (e.g. by comparing with HC2 protein level or mRNA level in a second biological sample).

Preferably, HC2 protein level or mRNA level in the first biological sample is measured or estimated and compared with a standard HC2 protein level or mRNA level, the standard being taken from a second biological sample obtained from an individual not having (nor presenting a susceptibility to) the disease. As will be appreciated, once a standard HC2 protein level or mRNA level is known, it can be used repeatedly as a standard for comparison.

By "biological sample" is intended any biological sample, either tissue sample or body fluid, obtained from an individual, cell line, tissue culture, or other source which contains HC2 protein or mRNA.

Total cellular RNA can be isolated from a biological sample using the single-step guanidinium-thiocyanate-phenol-chloroform method described in Chomczynski and Sacchi, Anal. Biochem. 162 : 156-159 (1987). Levels of mRNA encoding HC2 protein are then assayed using any appropriate method. These include Northern Blot analysis (Harada et al., Cell 63 : 303-312 (1990)), S1 nuclease mapping (Fujita et al., Cell 49 : 357-367 (1987)), the polymerase chain reaction (PCR), reverse transcription in combination with the polymerase chain reaction (RT-PCR) (Fujita et al., Cell 49 : 357-367 (1987)), and reverse transcription in combination with the ligase chain reaction (RT-LCR).

Assaying HC2 protein levels in a biological sample can occur using antibody-based techniques. For example, HC2 protein expression in tissues can be studied with classical immunohistological methods (Jalkanen, M., et al., J. Cell. Biol. 101 : 976-985 (1985); Jalkanen, M., et al., J. Cell. Biol. 105 : 3087-3096 (1987*))*. Other antibody-based methods useful for detecting HC2 gene expression include immunoassays, such as the enzyme linked immunosorbent assay (ELISA) and the radioimmunoassay (RIA).

Suitable labels are known in the art and include enzyme labels, such as glucose oxidase, and radioisotopes, such as iodine (¹²⁵I, ¹²¹I), carbon (¹⁴C), sulfur (³⁵S), tritium (³H), indium (¹¹²In), and technetium (^{99m}Tc), and fluorescent labels, such as fluorescein and rhodamine and biotin.

In addition, the invention provides a diagnostic assays for diagnosing or determining a susceptibility to cognitive disorders, developmental abnormalities and fetal deficiencies, neurodegenerative disorders such as amyotrophic lateral sclerosis, Parkinson's disease, schizophrenia, abnormal mental states, ischaemic shock, epilepsia, polyarthritis, hypertension, convulsions, ischaemic heart damage, ulcers, adrenal cortical function, wound healing, trauma, inflammatory diseases through detection of mutation in the HC2 gene by the methods described below.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled HC2 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers et al., Science (1985) 230:1242. Sequence changes at specific locations may also be revealed by nuclease protection assays, such as RNase and S1 protection or the chemical cleavage method. See Cotton et al., Proc. Natl. Acad. Sci. USA (1985) 85: 4397-4401. In another embodiment, an array of oligonucleotides probes comprising HC2 nucleotide sequence or fragments thereof can be constructed to conduct efficient screening of e.g., genetic mutations. Array technology methods are well known and have general applicability. They can be used to address a variety of questions in molecular genetics including gene expression, genetic linkage, and genetic variability. (See for example: M. Chee et al., Science, Vol 274, pp 610-613 (1996)).

The nucleotide sequences of the invention are also valuable for chromosome identification. The sequence is specifically targeted to and can hybridize with a particular location on an individual human chromosome. The mapping of relevant sequences to chromosomes according to the invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

The polypeptides of the invention or their fragments or analogs thereof, or cells expressing them can also be used as immunogens to produce antibodies or fragments of antibodies immunospecific for the HC2 polypeptides. The term "immunospecific" means that the antibodies have substantial greater affinity for the polypeptides of the invention than their affinity for other related polypeptides.

Antibodies generated against the HC2 polypeptides can be obtained by administering the polypeptides or epitope-bearing fragments, analogs or cells to an animal, preferably a non human, using routine protocols. For preparation of monoclonal antibodies, any technique which provides antibodies produced by continuous cell line cultures can be used. Examples include the hybridoma technique (Kohler, G. and Milstein, C., Nature (1975) 256:495-497), the trioma technique, the human B-cell hybridoma technique (Kozbor et al., Immunology Today (1983) 4:72) and the EBV-hybridoma technique (Cole et al., Monoclonal Antibodies and Cancer Therapy, pp. 77-96, Alan R. Liss, Inc., 1985).

Techniques for the production of single chain antibodies (U.S. Patent No. 4,946,778) can also be adapted to produce single chain antibodies to polypeptides of the invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against HC2 polypeptides may also be employed to treat cognitive disorders, developmental abnormalities and fetal deficiencies, neurodegenerative disorders such as amyotrophic lateral sclerosis, Parkinson's disease, schizophrenia, abnormal mental states, ischaemic shock, epilepsia, polyarthritis, hypertension, convulsions, ischaemic heart damage, ulcers, adrenal cortical function, wound healing, trauma, inflammatory diseases, among others.

Another aspect of the invention relates to a method for inducing an immunological response in a mammal which comprises inoculating the mammal with HC2 polypeptide, or a fragment thereof, adequate to produce antibody and/or T cell immune response to protect said mammal from cognitive disorders, developmental abnormalities and fetal deficiencies, neurodegenerative disorders such as amyotrophic lateral sclerosis, Parkinson's disease, schizophrenia, abnormal mental states, ischaemic shock, epilepsia, polyarthritis, hypertension, convulsions, ischaemic heart damage, ulcers, adrenal cortical function, wound healing, trauma, inflammatory diseases, among others. Yet another aspect of the invention relates to a method of inducing an immunological response in a mammal which comprises, delivering HC2 polypeptide via a vector directing expression of HC2 polynucleotide *in vivo* in order to induce such an immunological response and to produce antibodies to protect said mammal from diseases.

A further aspect of the invention relates to an immunological/vaccine formulation (composition) which, when introduced into a mammalian host, induces an immunological response in that mammal to a HC2 polypeptide wherein the composition comprises a HC2 polypeptide or HC2 gene. The vaccine formulation may further comprise a suitable carrier. Since HC2 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in-water systems and other systems known in the art. The dosage will depend on the specific activity of the vaccine and can be readily determined by routine experimentation.

The HC2 polypeptide of the invention may be employed in a screening process for candidate compounds which, directly or indirectly, stimulate (activators) or inhibit (inhibitors) the enzymatic activity of HC2. Thus, polypeptides of the invention may also be used to assess the binding of small molecule substrates and ligands in, for example, cells, cell-free preparations, chemical libraries, and natural product mixtures. These substrates and ligands may be natural substrates and ligands or may be structural or functional mimetics. See Coligan et al., Current Protocols in Immunology 1(2):Chapter 5 (1991).

The choice of the candidate compounds may be based on similarity with compounds known to interact with human carnosinase family, for example inhibitors or substrates of human carnosinase family. In particular, known inhibitors of the human carnosinase family may be used to determine candidate compounds to be screened.

Such known compounds include, but are not limited to, bestatine, phosphoramidon, 1,10-phenanthroline, p-hydroxymercuribenzoic acid, thiorphan.

Similarity of the candidate compounds with compounds known to activate or inhibit the enzymatic activity of the carnosinase family may be structural or functional, or both. For example, candidate compounds to be screened may be chosen starting from their chemical resemblance with known compounds. Other candidate compounds may be chosen by taking into account their functional relationship with the human carnosinase family.

Other candidate compounds can be chosen among compounds known to be involved in a large number of biological processes and can be selected for screening solely on this basis.

HC2 polypeptides are deemed to be responsible for many biological functions, including many pathologies. Accordingly, it is desirous to find compounds and drugs which stimulate HC2 on the one hand or which can inhibit the function of HC2 on the other hand. In general, such compounds are employed for therapeutic and prophylactic purposes for such conditions as cognitive disorders, developmental abnormalities and fetal deficiencies, neurodegenerative disorders such as amyotrophic lateral sclerosis, Parkinson's disease, schizophrenia, abnormal mental states, ischaemic shock, epilepsia, polyarthritis, hypertension, convulsions, ischaemic heart damage, ulcers, adrenal cortical function, wound healing, trauma, inflammatory diseases.

In general, such screening procedures involve producing appropriate cells which express the enzyme polypeptide of the invention on the surface thereof. Such cells include cells from mammals, yeast, *Drosophila* or *E. coli.* Cells expressing the enzyme (or cell membrane containing the expressed enzyme) are then contacted with a test compound to observe enzymatic activity, stimulation or inhibition of a functional response. Expressed enzyme can be purified to directly measure enzymatic activity.

Standard methods to measure enzymatic activity are well understood in the art. Such assays use various substrates and measure product formation. For instance, chromogenic or fluorogenic peptidic substrates are often used when the sequence of the natural substrate is known. Product formation is detected by measuring the release chromophore. Labeled proteins (like biotinylated hemoglobin) can be used to detect enzymatic activity with unknown substrate specificity. Lytic activity on an aspecific substrate like casein can be detected by clear lysed area in agarose gel containing casein.

Examples of potential HC2 inhibitors include antibodies or, in some cases, oligonucleotides or proteins which are closely related to the ligand of HC2, e.g., a fragment of the ligand, or small molecules which bind to the enzyme but do not elicit a response, so that the activity of the enzyme is prevented.

The invention also encompasses inhibitors or activators of HC2 identifiable by the methods described above.

The invention provides methods of treating abnormal conditions related to both an over-activity or an insufficient activity of HC2.

In a first aspect, the invention provides a method of treating an individual in need of an altered, diminished or inhibited level of HC2 activity or expression, comprising administering to such an individual a pharmaceutical composition comprising an effective amount of one or several active ingredients capable of altering or diminishing HC2 activity.

In that case, several approaches are available. One approach comprises administering as active ingredient an inhibitor compound identifiable by the method above described, optionally with a pharmaceutically acceptable carrier, in an amount effective to inhibit the enzymatic activity of HC2, or by inhibiting a second signal, and thereby alleviating the abnormal condition.

In another approach, soluble forms of HC2 polypeptides still capable of binding the substrate in competition with endogenous HC2 may be administered as active ingredient. Typical embodiments of such competitors comprise fragments of the HC2 polypeptide.

In still another approach, expression of the gene encoding endogenous HC2 can be inhibited using expression blocking techniques. Such known techniques involve the use of anti-sense sequences, either internally generated or separately administered. See, for example, O'Connor, J Neurochem (1991) 56:560 in Oligodeoxynucleotides as Anti-sense Inhibitors of Gene Expression, CRC Press, Boca Raton, FL (1988). Alternatively, oligonucleotides which form triple helices with the gene can be supplied. See, for example, Lee et al., Nucleic Acids Res. (1979) 6:3073; Cooney et al., Science (1988) 241:456; Dervan et al., Science (1991) 251:1360. These oligomers can be administered *per se* or the relevant oligomers can be expressed *in vivo*.

In a second aspect, the invention provides a method of treating an individual in need of an increased level of HC2 activity or expression, comprising administering to such an individual a pharmaceutical composition comprising an effective amount of one or several active ingredients capable of increasing HC2 activity.

In that case, several approaches are also available. One approach comprises administering as active ingredient a therapeutically effective amount of a compound which activates HC2, i.e., an activator identifiable by the method as described above, in combination with a pharmaceutically acceptable carrier, to thereby alleviate the abnormal condition.

Alternatively, gene therapy may be employed to effect the endogenous production of HC2 by the relevant cells in the subject. For example, a polynucleotide of the invention may be engineered for expression in a replication defective retroviral vector, as discussed above. The polynucleotide constitutes in that case the active ingredient. The retroviral expression construct may then be isolated and introduced into a packaging cell transduced with a retroviral plasmid vector containing RNA encoding a polypeptide of the invention such that the packaging cell now produces infectious viral particles containing the gene of interest. These producer cells may be administered to a subject for engineering cells *in vivo* and expression of the polypeptide *in vivo.* For overview of gene therapy, see Chapter 20, Gene Therapy and other Molecular Genetic-based Therapeutic Approaches, (and references cited therein) in Human Molecular Genetics, T Strachan and A. P. Read, BIOS Scientific Publishers Ltd (1996).

In another aspect, the invention provides pharmaceutical compositions for preventing and/or treating in a subject a disease or susceptibility to a disease related to activity of HC2.

Pharmaceutical compositions of the invention are intended, but not limited to, the treatment and/or prevention of cognitive disorders, developmental abnormalities and fetal deficiencies, neurodegenerative disorders such as amyotrophic lateral sclerosis, Parkinson's disease, schizophrenia, abnormal mental states, ischaemic shock, epilepsia, polyarthritis, hypertension, convulsions, ischaemic heart damage, ulcers, adrenal cortical function, wound healing, trauma, inflammatory diseases.

In a first aspect, the pharmaceutical compositions of the invention for preventing and/or treating in a subject a disease or susceptibility to a disease related to over-activity or over-expression of HC2 may comprise a therapeutically effective amount of at least an inhibitor of HC2 or a derivative or homologue thereof as defined above, and/or at least a nucleic acid molecule that inhibits the expression of the nucleotide sequence encoding said receptor (antisense sequence) and/or at least a peptide which competes with said receptor, as discussed above.

In a second aspect, the pharmaceutical compositions of the invention for preventing and/or treating in a subject a disease or susceptibility to a disease related to under-activity or under-expression of HC2 may comprise a therapeutically effective amount of at least an activator of HC2 or a derivative or homologue thereof as defined above, and/or at least a polynucleotide according to the invention and/or at least a nucleotide sequence according to the invention able to express *in vivo* a HC2 polypeptide of the invention.

The pharmaceutical compositions in which at least a polypeptide according to the invention is used may comprise the recombinant vectors of the invention, able to express *in vivo* a polypeptide according to the invention. Polypeptides according to the invention may be also used "naked", i.e. without being inserted into a vector. It has been shown that some DNA or RNA sequences can be expressed in some tissues without the aid of an expression vector.

Pharmaceutical compositions according to the invention, for example those which comprise polypeptides of the invention, such as the soluble form of HC2 polypeptides, activators, inhibitors or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, intrasternal, intraarterial or intraperitoneal, can be used. Alternative means for systemic administration include transmucosal and transdermal administration using penetrants such as bile salts or fusidic acids or other detergents. In addition, if properly formulated in enteric or encapsulated formulations, oral, rectal, intravaginal administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 1 µg/kg/day to 10 mg/kg/day of subject. More preferably, this dose is at least between 0.01 mg/kg/day and 1 mg/kg/day. Variations in the needed dosage are to be expected in view of the nature of the compounds used and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide of the invention, such as a DNA or RNA, to encode a polypeptide *ex vivo*, and for example, by the use of a retroviral plasmid vector. The cells are then introduced into the subject.

The examples below are carried out using standard techniques, which are well known and routine to those of skill in the art, except where otherwise described in detail. The examples illustrate, but do not limit the invention.

### Example 1.

### Cloning of human carnosinase 2.

The sequence of HC2 is first identified by searching a database containing approximately 2 millions human ESTs, which was generated using high throughput automated DNA sequence analysis of randomly selected human cDNA clones (Adams, M.D. et al., Nature 377:3-174 (1995); Adams, M.D. et al., Nature 355:632-634 (1992); and Adams, M.D. et al., Science 252:1651-1656 (1991)). Sequence homology comparisons of each EST are performed against the GenBank database using the blastn and tblastn algorithms (Altschul, S.F. et al., J. Mol. Biol. 215:403-410 (1990)). No homologous human sequences were identified. One homologous sequence - yeast hypothetical 52.9 KD protein - shows around 66 % similarity with HC2. Electronic analysis of the distribution of the EST reveals its ubiquitous localisation. Sequence of the gene is confirmed by double strand DNA sequencing and the gene is shown to be completely new by a blast search against Genbank.

### Example 2.

### Cloning and expression of HC2 in mammalian cells.

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as PSVL and PMSG (Pharmacia, Uppsala, Sweden), pRSVcat (ATCC 37152), pSV2dhfr (ATCC 37146) and pBC12Ml (ATCC 67109) and pcDNA3 (Invitrogen). Mammalian host cells that could be used include, human HEK 293, H9 and Jurkat cells, mouse NIH3T3 and C127 cells, Cos 1, Cos 7 and mouse L cells and Chinese hamster ovary (CHO) cells.

Alternatively, the gene can be expressed in stable cell lines that contain the gene integrated into a chromosome. The co-transfection with a selectable marker such as dhfr, gpt, neomycin, zeocin or hygromycin allows the identification and isolation of the transfected cells.

The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy et al., Biochem. J. 227:277-279 (1991); Bebbington et al., Bio/Technology 10:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

The expression vector pCDNA3 (Invitrogen) contains the strong promoter (CMV) of the Cytomegalovirus. Multiple cloning sites allow the cloning of foreign genes. The entire coding region of HC2 is inserted into the expression vector pCDNA3(-) by use of *EcoR1* and *BamHI* restriction enzymes. The presence of the insert and its orientation in the vector is detected by restriction analysis.

The expression vector carrying HC2 is used to transfect CHO cells, using standard protocols. Expression of HC2 polypeptide is detected by measurement of carnosinase activity. Cell lysates and cell supernatants are analysed.

### Example 3.

### Master blot.

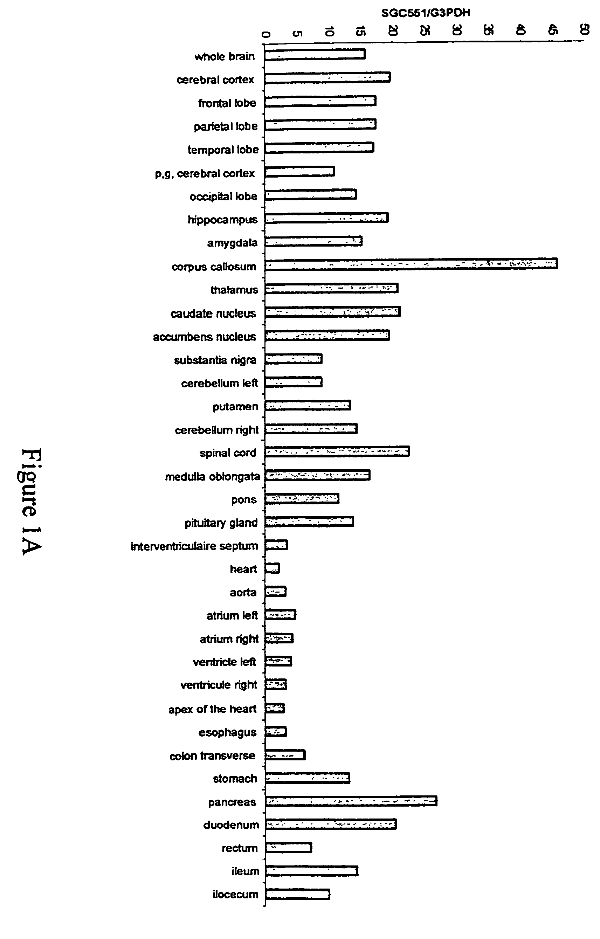

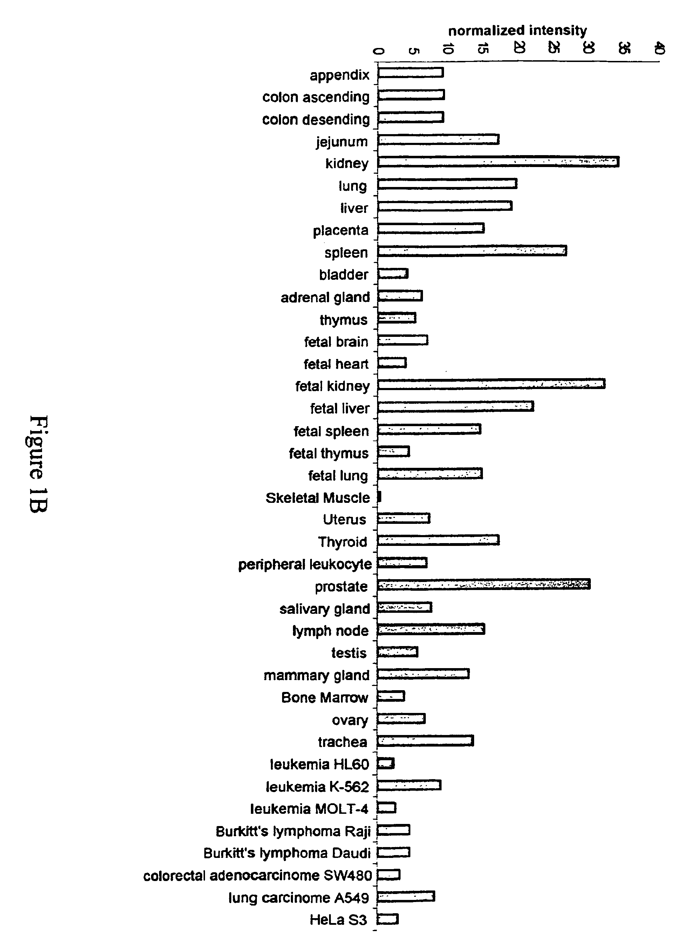

Tissue distribution of HC2 is determined by hybridization of the cDNA probe obtained from the HC2 clone by *EcorI-KpnI* digestion. The probe is radiolabeled with [32P]dCTP. This probe is used to hybridize a Human RNA Master Dot blot (Clontech, ref 7770-1) with 50 different tissues represented (100-500 ng / lane) in Express Hyb buffer (Clontech), according to the manufacturer specifications. The signal is detected using a Storm (Molecular Dynamics). Results show that HC2 mRNA is expressed in all human tissues (Figure 1).

### Example 4.

### Functional activity of Human Carnosinase by in vitro measurements.

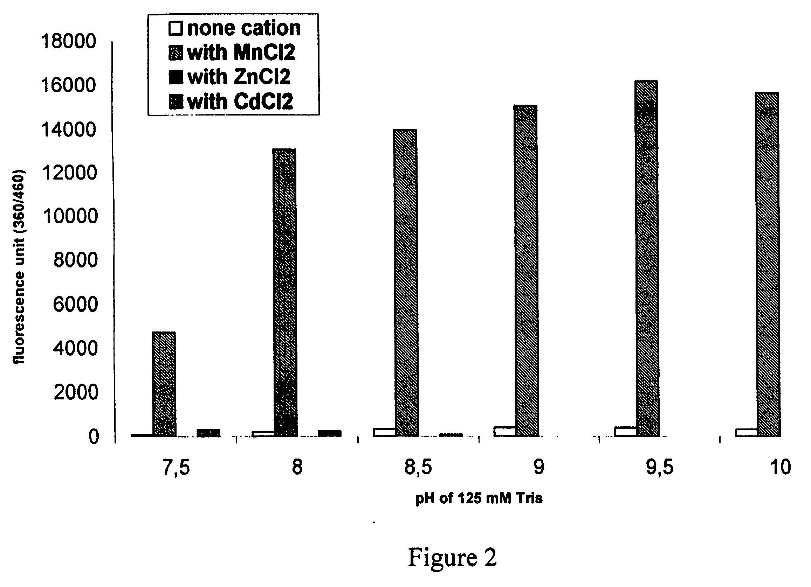

Experiments are performed on transiently transfected CHO cells obtained using standard techniques. Protocols for tissue carnosinase are applied. Cells transfected with HC2 cDNA are diluted in 10 mM Tris-HCI pH 7.5. Cells are sonicated 3 times for 10 sec. on ice and centrifuged 15,000 g / 10 min. at 4°C. Enzymatic assay is performed in black 96-well plate. 5 µl of sample are mixed with 15 µl of buffer A (125 mM Tris pH : 9.5 in presence of 0.083 mM MnCl₂ in 150 mM NaCl) and incubated at room temperature for 15 min. 5 µl of substrate (60 mM carnosine in 125 mM Tris-HCI, pH 9.5) are added and incubated at 32°C for 60 min. The reaction is stopped by precipitation with 15 µl of 0.6N trichloroacetic acid. Measurement of Histidine is performed after precipitation of 30 µl of supernatant by addition of 100 µl of 1N NaOH and 50 µl of OPT (50 mg o-phthalaldehyde / 100 ml ethanol 95%), incubation at 32°C for 15 min., addition of 50 µl of buffer B (56.36 g P₂O₇ K₄.10H₂O + 1.65 g HNa₂O₃S/1 liter 8N phosphoric acid), incubation for 15 min performed at room temperature. Finally, the plate is agitated and reading of the fluorescence is performed at room temperature (excitation 360 nm, emission 460 nm). Results appear in Figure 2. Carnosinase activity is found exclusively in the cytosol of transfected cells. Optimum of pH and cation specificity are : pH 9.5; MnCl₂.

## Claims

1. A polynucleotide comprising a nucleotide sequence at least 80% identical to a nucleotide sequence selected from the group consisting of:
(a) the nucleotide sequence set forth in SEQ ID NO:1;
(b) a nucleotide sequence which comprises the human carnosinase 2 HC2 polypeptide encoding sequence contained in SEQ ID NO:1;
(c) a nucleotide sequence comprising the sequence located between nucleotide numbers 1 to 1425 set forth in SEQ ID NO:1;
(d) a nucleotide sequence encoding a polypeptide comprising amino acids from 1 to 475 set forth in SEQ ID NO:2;
(e) the nucleotide sequence encoding the HC2 polypeptide expressed by the cDNA clone contained in ATCC deposit number PTA-1045;
(f) the nucleotide sequence encoding the mature HC2 polypeptide expressed by the cDNA clone contained in ATCC deposit number PTA-1045;
(g) a nucleotide sequence able to hybridize under stringent conditions with the nucleotide sequence set forth in SEQ ID NO:1 or with the cDNA clone contained in ATCC deposit number PTA-1045;
(h) a nucleotide sequence complementary to any of the nucleotide sequences defined in (a), (b), (c), (d), (e), (f) or (g) above, as well as fragments forms of these nucleotide sequences encoding a protein that mediates HC2 activity,
with the proviso that the polynucleotide has not one of the following sequences: and

2. Polynucleotide according to claim 1 which is DNA or RNA.

3. A DNA or RNA molecule constituting a recombinant vector, comprising an expression system which is capable of producing a HC2 polypeptide when said expression system is present in a compatible host cell, wherein said DNA or RNA molecule comprises a polynucleotide according to claim 1 or 2.

4. A isolated host cell comprising the expression system of claim 3.

5. A process for producing a HC2 polypeptide, comprising the steps of transforming or transfecting a host cell with an expression system according to claim 3, culturing said host cell under appropriate culture conditions so that said host cell produces the HC2 polypeptide, and recovering the polypeptide from the culture.

6. A HC2 polypeptide comprising an amino add sequence which is at least 80% identical to a sequence selected from the group consisting of:
(a) the amino acid sequence set forth in SEQ ID NO:2;
(b) the amino acid sequence of the HC2 polypeptide having the complete amino acid sequence encoded by the cDNA clone contained in ATCC deposit number PTA-1045;
(c) the amino add sequence of the mature HC2 polypeptide having the complete amino acid sequence encoded by the cDNA clone contained in ATCC deposit number PTA-1045; as well as fragments that mediates HC2 activity or, variants which show substantial HC2 polypetide activity of these amino acid sequences.

7. Polypeptide according to claim 6, wherein it is obtained by the process of claim 5 or by chemical synthesis.

8. An antibody, or a fragment thereof, able to recognize a polypeptide according to claim 6.

9. Antibody according to claim 8, able to recognize a polypeptide comprising amino acid residues from 10 to 25 of the amino add sequence set forth in SEQ ID NO:2, a polypeptide comprising amino acid residues from 445 to 460 of the amino acid sequence set forth in SEQ ID NO:2.

10. A method for identifying activators of HC2 polypeptide, which comprises the steps of :
(a) contacting a host cell according to claim 4 with a candidate compound; and
(b) determining whether the candidate compound effects a signal generated by activation of the HC2 polypeptide.

## Patentansprüche

1. Polynukleotid umfassend eine Nukleotidsequenz mit mindestens 80% Identität zu einer Nukleotidsequenz ausgewählt aus der Gruppe bestehend aus:
(a) der Nukleotidsequenz gemäß SEQ ID NO:1;
(b) einer Nukleotidsequenz, die die in SEQ ID NO:1 enthaltene Codiersequenz für das Polypeptid für die humane Carnosinase 2 (HC2) umfaßt;
(c) einer Nukleotidsequenz, die die Sequenz, die sich zwischen den Nukleotiden Nummer 1 bis 1425 gemäß SEQ ID NO:1 befindet, umfaßt;
(d) einer Nukleotidsequenz, die für ein Polypeptid umfassend die Aminosäuren von 1 bis 475 gemäß SEQ ID NO:2 codiert;
(e) der Nukleotidsequenz, die für das HC2-Polypeptid, das von dem in ATCC-Hinterlegungsnummer PTA-1045 enthaltenen cDNA-Klon exprimiert wird, codiert;
(f) der Nukleotidsequenz, die für das reife HC2-Polypeptid, das von dem in ATCC Hinterlegungsnummer PTA-1045 enthaltenen cDNA-Klon exprimiert wird, codiert;
(g) einer Nukleotidsequenz, die fähig ist, mit der Nukleotidsequenz gemäß SEQ ID NO:1 oder mit dem in ATCC-Hinterlegungsnummer PTA-1045 enthaltenen cDNA-Klon unter stringenten Bedingungen zu hybridisieren;
(h) einer Nukleotidsequenz, die zu einer der in (a), (b), (c), (d), (e), (f) oder (g) oben definierten Nukleotidsequenzen komplementär ist, sowie Fragmentformen dieser Nukleotidsequenzen, die für ein Protein, welches HC2-Aktivität vermittelt, codieren,
mit der Maßgabe, daß das Polynukleotid nicht eine der folgenden Sequenzen aufweist: und

2. Polynukleotid nach Anspruch 1, bei dem es sich um DNA oder RNA handelt.

3. DNA- oder RNA-Molekül, das einen rekombinanten Vektor bildet, umfassend ein Expressionssystem, das fähig ist, bei Vorliegen dieses Expressionssystems in einer kompatiblen Wirtszelle ein HC2-Polypeptid zu produzieren, wobei das DNA- oder RNA-Molekül ein Polynukleotid nach Anspruch 1 oder 2 umfaßt.

4. Isolierte Wirtszelle umfassend das Expressionssystem nach Anspruch 3.

5. Verfahren zur Herstellung eines HC2-Polypeptids, umfassend die Schritte: Transformieren oder Transfizieren einer Wirtszelle mit einem Expressionssystem nach Anspruch 3, Kultivieren dieser Wirtszelle unter geeigneten Kulturbedingungen, so daß die Wirtszelle das HC2-Polypeptid produziert, und Gewinnen des Polypeptids aus der Kultur.

6. HC2-Polypeptid umfassend eine Aminosäuresequenz mit mindestens 80% Identität zu einer Sequenz ausgewählt aus der Gruppe bestehend aus:
(a) der Aminosäuresequenz gemäß SEQ ID NO:2;
(b) der Aminosäuresequenz des HC2-Polypeptids mit der vollständigen Aminosäuresequenz, die von dem in ATCC-Hinterlegungsnummer PTA-1045 enthaltenen cDNA-Klon codiert wird;
(c) der Aminosäuresequenz des reifen HC2-Polypeptids mit der vollständigen Aminosäuresequenz, die von dem in ATCC-Hinterlegungsnummer PTA-1045 enthaltenen cDNA-Klon codiert wird; sowie Fragmente dieser Aminosäuresequenzen, die HC2-Aktivität vermitteln, oder Varianten dieser Aminosäuresequenzen mit im wesentlichen HC2-Polypeptidaktivität.

7. Polypeptid nach Anspruch 6, das durch das Verfahren nach Anspruch 5 oder durch chemische Synthese erhalten wird.

8. Antikörper oder Fragment davon, der/das fähig ist, ein Polypeptid nach Anspruch 6 zu erkennen.

9. Antikörper nach Anspruch 8, der fähig ist, ein Polypeptid mit Aminosäureresten von 10 bis 25 der Aminosäuresequenz gemäß SEQ ID NO:2, ein Polypeptid mit Aminosäuren von 445 bis 460 der Aminosäuresequenz gemäß SEQ ID NO:2 zu erkennen.

10. Verfahren zum Identifizieren von Aktivatoren des HC2-Polypeptids, das die folgenden Schritte umfaßt:
(a) Inkontaktbringen einer Wirtszelle nach Anspruch 4 mit einer Kandidatenverbindung; und
(b) Bestimmen, ob die Kandidatenverbindung ein durch Aktivierung des HC2-Polypeptids erzeugtes Signal bewirkt.

## Revendications

1. Polynucléotide comprenant une séquence nucléotidique au moins 80 % identique à une séquence nucléotidique choisie dans le groupe constitué :
(a) de la séquence nucléotidique exposée dans SEQ ID NO : 1 ;
(b) d'une séquence nucléotidique qui comprend la séquence codant pour le polypeptide de la carnosinase 2 humaine HC2 contenue dans SEQ ID NO : 1 ;
(c) d'une séquence nucléotidique comprenant la séquence située entre les nucléotides numéros 1 à 1425 exposés dans SEQ ID NO : 1 ;
(d) d'une séquence nucléotidique codant pour un polypeptide comprenant les acides aminés 1 à 475 exposés dans SEQ ID NO : 2 ;
(e) de la séquence nucléotidique codant pour le polypeptide HC2 exprimée par le clone d'ADNc contenu dans le dépôt ATCC numéro PTA-1045 ;
(f) de la séquence nucléotidique codant pour le polypeptide HC2 mature exprimée par le clone d'ADNc contenu dans le dépôt ATCC numéro PTA-1045 ;
(g) d'une séquence nucléotidique capable de s'hybrider dans des conditions stringentes avec la séquence nucléotidique exposée dans SEQ ID NO : 1 ou avec le clone d'ADNc contenu dans le dépôt ATCC numéro PTA-1045 ;
(h) d'une séquence nucléotidique complémentaire de l'une quelconque des séquences nucléotidiques définies en (a), (b), (c), (d), (e), (f) ou (g) ci-dessus, ainsi que des formes de fragments de ces séquences nucléotidiques codant pour une protéine qui médie l'activité d'HC2,
à condition que le polynucléotide n'ait pas l'une des séquences suivantes : et

2. Polynucléotide selon la revendication 1, qui est de l'ADN ou de l'ARN.

3. Molécule d'ADN ou d'ARN constituant un vecteur recombinant, comprenant un système d'expression qui est capable de produire un polypeptide HC2 lorsque ledit système d'expression est présent dans une cellule hôte compatible, ladite molécule d'ADN ou d'ARN comprenant un polynucléotide selon la revendication 1 ou 2.

4. Cellule hôte isolée comprenant le système d'expression de la revendication 3.

5. Procédé de production d'un polypeptide HC2, comprenant les étapes consistant à transformer ou à transfecter une cellule hôte avec un système d'expression selon la revendication 3, à mettre ladite cellule hôte en culture dans des conditions de culture appropriées de façon à ce que ladite cellule hôte produise le polypeptide HC2 et à récupérer le polypeptide à partir de la culture.

6. Polypeptide HC2 comprenant une séquence d'acides aminés qui est au moins 80 % identique à une séquence choisie dans le groupe constitué :
(a) de la séquence d'acides aminés exposée dans SEQ ID NO : 2 ;
(b) de la séquence d'acides aminés du polypeptide HC2 possédant la séquence d'acides aminés entière codée par le clone d'ADNc contenu dans le dépôt ATCC numéro PTA-1045 ;
(c) de la séquence d'acides aminés du polypeptide HC2 mature possédant la séquence d'acides aminés entière codée par le clone d'ADNc contenu dans le dépôt ATCC numéro PTA-1045 ; ainsi que des fragments, qui médient l'activité d'HC2, ou des variants, qui présentent une activité de polypeptide HC2 sensible, de ces séquences d'acides aminés.

7. Polypeptide selon la revendication 6, **caractérisé en ce qu'**il est obtenu par le procédé de la revendication 5 ou par synthèse chimique.

8. Anticorps, ou fragment de celui-ci, capable de reconnaître un polypeptide selon la revendication 6.

9. Anticorps selon la revendication 8, capable de reconnaître un polypeptide comprenant les résidus d'acides aminés des positions 10 à 25 de la séquence d'acides aminés exposée dans SEQ ID NO : 2, un polypeptide comprenant les résidus d'acides aminés des positions 445 à 460 de la séquence d'acides aminés exposée en SEQ ID NO : 2.

10. Méthode d'identification d'activateurs du polypeptide HC2, qui comprend les étapes consistant à :
(a) mettre une cellule hôte selon la revendication 4 en contact avec un composé candidat ; et
(b) déterminer si le composé candidat entraîne un signal généré par l'activation du polypeptide HC2.
